# EUROPEAN PATENT SPECIFICATION

(11) **EP 4 452 981 B1**
(45) Date of publication and mention of the grant of the patent: **27.08.2025**
(21) Application number: 22843266.2
(22) Date of filing: 21.12.2022
(51) Int. Cl.: C07D 487/04, A61K 31/519, A61P 11/00

(54) **TRIAZOLONE DERIVATIVE SALT AS NEUTROPHIL ELASTASE INHIBITOR**
TRIAZOLONDERIVATSALZ ALS INHIBITOR DER NEUTROPHILEN ELASTASE
SEL DÉRIVÉ DE TRIAZOLONE EN TANT QU'INHIBITEUR DE L'ÉLASTASE DU NEUTROPHILE

(30) Priority: 22.12.2021 EP 21217142
(43) Date of publication of application: 30.10.2024
(73) Proprietor: Chiesi Farmaceutici S.p.A., 43122 Parma (IT)
(72) Inventor: AMARI, Gabriele, 43122 Parma (IT); CAPALDI, Carmelida, 43122 Parma (IT); ARMANI, Elisabetta, 43122 Parma (IT)
(74) Representative: Chiesi Farmaceutici S.p.A.
(86) International application number: PCT/EP2022/087169
(87) International publication number: WO 2023/118258

(56) References cited:
- WO-A1-2014/095700

## Description

### Field of the invention

The present invention generally relates to a novel triazolone derivative salt particularly useful as neutrophil elastase inhibitor and to its use as a medicament. The present invention also relates to pharmaceutical compositions comprising the novel triazolone derivative salt and one or more pharmaceutically acceptable carriers and/or excipients thereof and to their use as medicament.

### Background of the invention

Human neutrophil elastase (HNE) is a 32 kDa serine proteinase found in the azurophilic granules of neutrophils. It has a role in the degradation of a wide range of extracellular matrix proteins, including fibronectin, laminin, proteoglycans, Type III and Type IV collagens as well as elastin (Bieth, G. In Regulation of Matrix accumulation, Mecham, R.P. (Eds), Academic Press, NY, USA 1986, 217-306). HNE has long been considered to play an important role in homeostasis through repair and disposal of damaged tissues via degradation of the tissue structural proteins. It is also relevant in the defense against bacterial invasion by means of degradation of the bacterial body. In addition to its effects on matrix tissues, HNE has been implicated in the upregulation of IL-8 gene expression and also induces IL-8 release from the epithelial cells of the lung. In animal models of Chronic Obstructive Pulmonary Disease induced by tobacco smoke exposure both small molecule inhibitors and protein inhibitors of HNE inhibit the inflammatory response and the development of emphysema (Wright, J.L. et al. Am. J. Respir. Crit. Care Med. 2002, 166, 954-960; Churg, A. et al. Am. J. Respir. Crit. Care Med. 2003, 168, 199-207). Thus, HNE may play a role both in matrix destruction and in amplifying inflammatory responses in chronic respiratory diseases where neutrophil influx is a characteristic feature. Indeed, HNE is believed to play a role in several pulmonary diseases, including chronic obstructive pulmonary disease (COPD), cystic fibrosis (CF), acute respiratory distress syndrome (ARDS), pulmonary emphysema, pneumonia and lung fibrosis. It is also implicated in several cardiovascular diseases in which tissue remodelling is involved, for example, in heart failure and the generation of ischaemic tissue injury following acute myocardial infarction.

Excess HNE activity has been implicated in the pathology of inflammatory pulmonary diseases, including bronchiectasis (BE), so identifying this protein as a target for drug development (B. Schaaf, A. Wieghorst, S.-P. Aries, K. Dalhoff, J. Braun, Respiration, 67 (1) (2000), 52-59).

Several human neutrophil inhibitors have been disclosed so far in the art.

In particular, the International Patent Applications n. WO2011/110858 and n. WO2011/110859 describe some pyrimidine derivatives having human neutrophil elastase inhibitory properties and their use in therapy.

WO 2014/095700 describes triazolone derivatives having human neutrophil elastase inhibitory properties and their use in therapy and, in particular, some salts of the compound (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium as potent neutrophil elastase inhibitors.

Although several HNE inhibitors have been disclosed so far as above reported, there is still a need for further HNE inhibitors.

Particularly advantageous would be the identification of further efficacious HNE inhibitors endowed with a safer profile, suitable for administration, also in terms of patients tolerability and local adverse effects profile.

The present invention addresses the above mentioned needs by providing the compound of the invention, (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium hemi-pamoate of formula (I).

### Summary of the invention

In a first aspect, the present invention provides (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I)

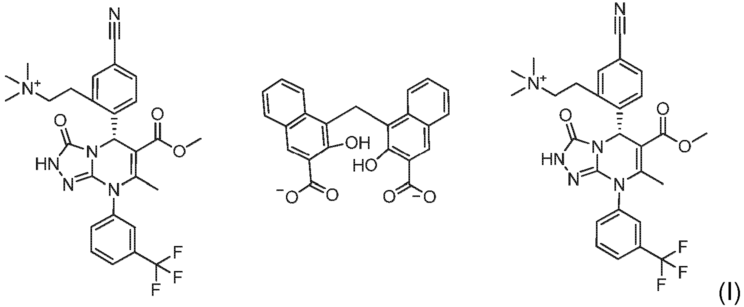

In a second aspect, the invention provides a pharmaceutical composition comprising (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I) and one or more pharmaceutically acceptable carriers and/or excipients.

In a further aspect, the invention provides (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I) for use as a medicament.

In a still further aspect, the invention provides a pharmaceutical composition comprising (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I) and one or more pharmaceutically acceptable carriers and/or excipients for use as a medicament.

In another aspect, the disclosure provides (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium hemi-pamoate of formula (I) in the manufacture of a medicament for the prevention and/or treatment of an inflammatory or obstructive respiratory disease.

In a further aspect, the disclosure provides a pharmaceutical composition comprising (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I) and one or more pharmaceutically acceptable carriers and/or excipients in the manufacture of a medicament for the prevention and/or treatment of an inflammatory or obstructive respiratory disease.

In another aspect, the present disclosure provides a method for preventing and/or treating an inflammatory or obstructive respiratory disease, the method comprising administering an effective amount of (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I).

In a further aspect, the present disclosure provides a method for preventing and/or treating an inflammatory or obstructive respiratory disease, the method comprising administering an effective amount of pharmaceutical composition comprising (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I) and one or more pharmaceutically acceptable carriers and/or excipients.

In another aspect, the present invention provides a process for the preparation of the compound of formula (I), by reacting the triazolone derivative of formula (II) with a pamoate salt of formula (III) 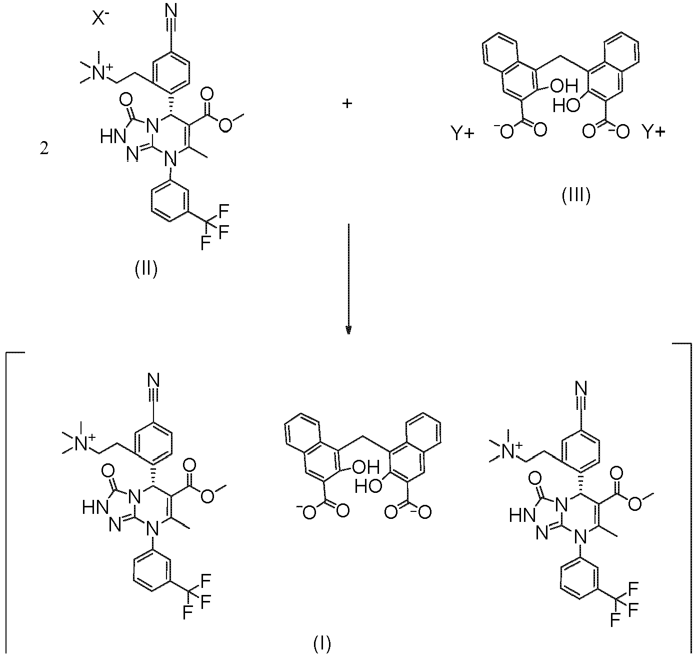 said process comprising the steps of:
1) dissolving in water a water-soluble salt of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium of formula (II): 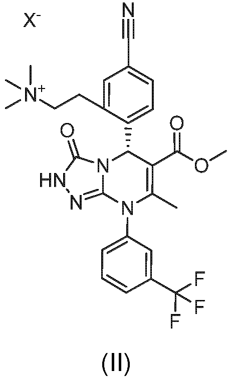 wherein X⁻ is an organic or inorganic anion, and
2) adding a solution of a pamoate salt of formula (III) 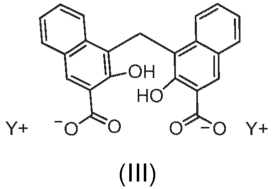 wherein Y⁺ is an alkaline or alkaline earth metal cation.

### Detailed Description of the Invention

### DEFINITIONS

Unless defined otherwise, all technical and scientific terms used herein have the same meanings as commonly understood by the skilled in the art.

The term "compound of the invention" refers to (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I).

The term "hemi-pamoate" refers to the salt with pamoate di-anion in which the stoichiometric ratio between 2-{5-Cyano-2-[(R)-6-methoxycarbonyl-7-methyl-3-oxo-S-(3-trifluoromethyl-phenyl)-2,3,5,8-tetrahydro-[1,2,4]triazolo[4,3-a]pyrimidin-5-yl]-phenyl}-ethyl)-trimethyl-ammonium cation and pamoate di-anion is 2:1.

The terms "alkaline or alkaline earth metal cation" are intended to indicate cations of metal elements selected from sodium, potassium, magnesium and calcium.

The compound of the invention has one stereogenic center, namely represented by the carbon atom (1) with an asterisk * below and therefore can exist as optical stereoisomers

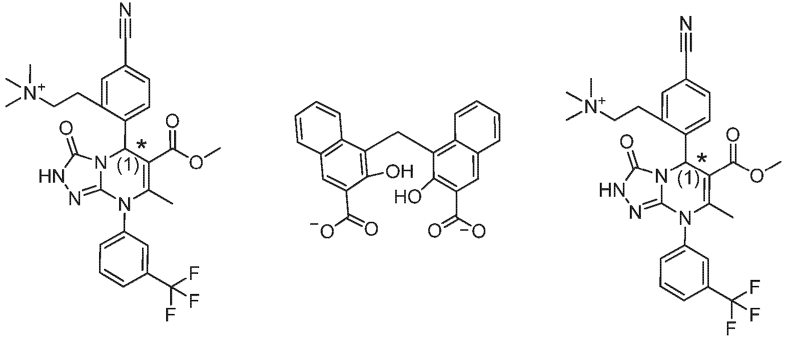

It is to be understood that besides the compound of the invention of formula (I) showing a preferred (R) configuration on carbon atom (1), the racemic form and enantiomer (S) are encompassed within the present invention

The term "composition", as in pharmaceutical composition, is intended to encompass a product comprising the active ingredient and any pharmaceutically acceptable excipient or carrier, as well as any product which results, directly or indirectly, from combination, complexation or aggregation of any two or more of the ingredients, or from dissociation of one or more of the ingredients, or from other types of reactions or interactions of one or more of the ingredients.

Accordingly, the pharmaceutical compositions of the invention encompass any composition made by admixing the compound of the invention and pharmaceutically acceptable excipients and/or carriers.

The hemi-pamoate salt as indicated in formula (I), is characterized by having physico-chemical features particularly suitable for administration, also in terms of patients tolerability and local adverse effects profile.

Advantageously, in this respect, the hemi-pamoate salt according to compound of formula (I) of the invention shows:
1) an unexpected, considering the lower solubility with respect to methanesulfonate salt, superior activity in a pharmacodynamic model, with respect to the methanesulfonate salt, when administered as dry powder, as described in example 2.1 of the Biological assay and
2) a very good irritative profile in the HOP (Head Out Plethysmography) test in conscious rats, in comparison with the methanesulfonate, bromide and propionate salts, when administered as dry powder, thus suggesting a possible safer profile also in humans, as described and commented in the present example 2.2.

In consideration of the above, the hemi-pamoate salt of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium maintains, despite its lower solubility with respect to the methanesulfonate salt, a good efficacy in inhibiting HNE in-vivo and maintains a good efficacy also when administered as dry powder formulation.

Moreover, the Head-out plethysmography (HOP) assay showed that conversely to the other salts methanesulfonate, bromide and propionate of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium, the hemi-pamoate salt of formula (I), shows improved lung function parameters, suggesting that this salt is particularly suitable for administration, also in terms of patients tolerability and local adverse effects profile.

The present invention is also directed to a pharmaceutical composition comprising the compound of formula (I) and one or more pharmaceutically acceptable carriers and/or excipients.

Suitable excipients can be selected among those in the art, and they can include carriers, diluents, wetting agents, emulsifying agents, binders, coatings, fillers, glidants, lubricants, disintegrants, preservatives, surfactants, pH buffering substances and the like. Examples of excipients and their use are provided in the Handbook of Pharmaceutical Excipients, 5th ed. (2006), Ed. Rowe et al., Pharmaceutical Press.

The most suitable dosage level may be determined by any known suitable method. It will be understood, however, that the specific amount for any particular patient will depend upon a variety of factors, including the activity of the compound of formula (I), the age, body weight, diet, general health and sex of the patient, time of administration, the route of administration, the rate of excretion, the use of any other drugs, and the severity of the disease to be treated.

For delivery by inhalation, the active compound of formula (I) is preferably in the form of microparticles. They may be prepared by a variety of techniques, including spray-drying, freeze-drying and micronisation.

In one embodiment, a composition of the invention is prepared as a suspension, suitable for delivery from a nebuliser or as an aerosol in a liquid propellant, even more preferably for use in a pressurised metered dose inhaler (PMDI). Suitable propellants for use in a pMDI are known to the skilled person, and include HFA-227, preferably HFA-134a and more preferably HFA152a.

In a preferred embodiment, a composition of the invention is in dry powder form, for delivery using a dry powder inhaler (DPI).

Microparticles for delivery by administration may be formulated with excipients that aid delivery and release. For example, in a dry powder formulation, microparticles may be formulated with large carrier particles that aid flow from the DPI into the lung. Suitable carrier particles are known in the art and include e.g. lactose particles.

The agents of the invention may be administered in inhaled form. Aerosol generation can be carried out using, for example, pressure-driven jet atomizers or ultrasonic atomizers, preferably using propellant-driven metered aerosols or propellant-free administration of micronised active compound of formula (I) from, for example, inhalation capsules or other "dry powder" delivery systems.

As above described, the present invention is directed to the compound of general formula (I) for use as a medicament.

According to a preferred embodiment, the present invention refers to the use of the hemi-pamoate salt of formula (I) for the preparation of a medicament for the treatment of an inflammatory or obstructive pulmonary disease, preferably selected from: : asthma, chronic obstructive pulmonary disease (COPD), bronchiectasis, chronic bronchitis, lung fibrosis, idiopathic pulmonary fibrosis, pneumonia, acute respiratory distress syndrome (ARDS), pulmonary emphysema, smoking-induced emphysema and cystic fibrosis.

The present invention is also directed to a pharmaceutical composition comprising the compound of formula (I) and one or more pharmaceutically acceptable carriers and/or excipients for use as a medicament.

In a preferred embodiment, the present invention is directed to the compound of formula (I) for use for the prevention and/or treatment of an inflammatory or obstructive respiratory disease.

In another preferred embodiment, the present invention is directed to a pharmaceutical composition comprising the compound of formula (I) and one or more pharmaceutically acceptable carriers and/or excipients, for use for the prevention and/or treatment of an inflammatory or obstructive respiratory disease.

In a further preferred embodiment, the present disclosure provides a method for preventing and/or treating an inflammatory or obstructive respiratory disease, the method comprising administering an effective amount of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I).

In another preferred embodiment, the present disclosure provides a method for preventing and/or treating an inflammatory or obstructive respiratory disease, the method comprising administering an effective amount of pharmaceutical composition comprising (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I) and one or more pharmaceutically acceptable carriers and/or excipients.

In a still further preferred embodiment, the inflammatory or obstructive respiratory diseases mentioned above are selected from: asthma, chronic obstructive pulmonary disease (COPD), bronchiectasis, chronic bronchitis, lung fibrosis, idiopathic pulmonary fibrosis, pneumonia, acute respiratory distress syndrome (ARDS), pulmonary emphysema, smoking-induced emphysema and cystic fibrosis.

Any suitable route of administration may be employed for providing a mammal, especially a human, with an effective dosage of the compound of formula (I).

The magnitude of prophylactic or therapeutic dose of the compound of formula (I) will, of course, vary with the nature of the severity of the condition to be treated and with its route of administration, and will generally be determined by clinical trial as required in the pharmaceutical art.

It will also vary according to the age, weight and response of the individual patient.

In therapeutic use, the compound of formula (I) may be administered by any convenient, suitable or effective route.

Suitable routes of administration are known, and include oral, intravenous, rectal, parenteral, topical, ocular, nasal, buccal and pulmonary (by inhalation).

The active compound of formula (I) may be dosed as described depending on the inhaler system used. In addition to the active compound, the administration forms may additionally contain excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g. Nebulator^{®}, Volumatic^{®}), and automatic devices emitting a puffer spray (Autohaler^{®}), for metered aerosols, in particular in the case of powder inhalers, a number of technical solutions are available (e.g. Diskhaler^{®}, Rotadisk^{®}, Turbohaler^{®} or the inhalers for example as described EP-A-0505321).

As aforementioned, the present invention also refers to a process for the preparation of the compound of formula (I), by reacting the triazolone derivative of formula (II) with a pamoate salt of formula (III) as above identified, said process comprising the steps of:
1) dissolving in water a water soluble salt of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium of formula (II) wherein X⁻ is an organic or inorganic anion, and
2) adding a solution in water of a pamoate salt of formula (III) wherein Y⁺ is an alkaline or alkaline earth metal cation.

In another preferred embodiment, the present invention provides a process for the preparation of a compound of formula (I) by reacting a compound of formula (II), wherein X⁻ is bromide.

In another preferred embodiment, the present invention provides a process for the preparation of the compound of formula (I), by reacting a compound of formula (II), wherein X⁻ is methanesulfonate.

In a further preferred embodiment, the present invention provides a process for the preparation of the compound of formula (I), by reacting a pamoate salt of formula (III), wherein Y⁺ is sodium or potassium, being sodium even more preferred.

In a still further preferred embodiment, the present invention also provides a process for the preparation of the compound of formula (I), by reacting a compound of formula (II) with a pamoate salt of formula (III), wherein the molar ration between (II) and (III) is 2:1.

In a more preferred embodiment, a process for the preparation of the compound of the invention of formula (I) wherein the stoichiometric ratio between 2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium cation and pamoate di-anion is 2:1, is provided, according to general synthetic route reported in **Scheme A** here below 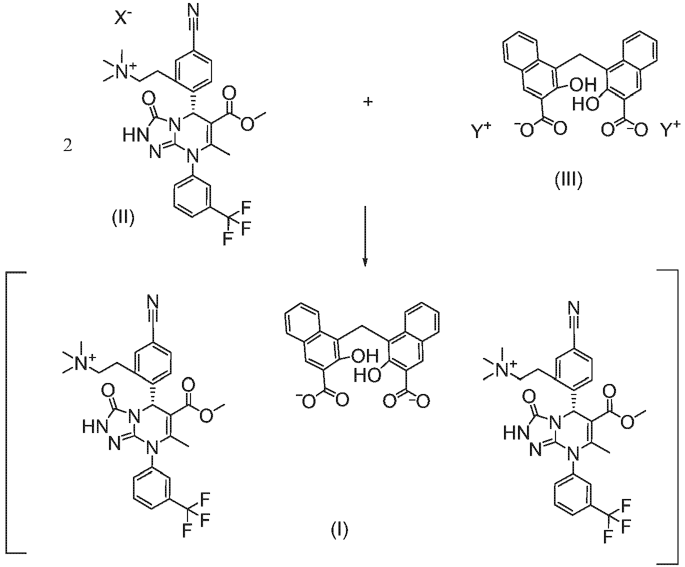 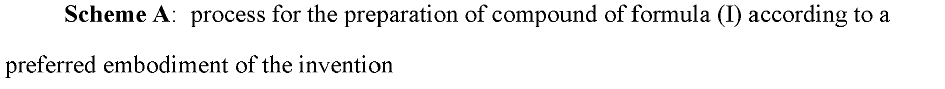 wherein X⁻ is an organic or inorganic anion, preferably selected from the group consisting of methanesulfonate, propionate and bromide and Y⁺ is an alkaline or alkaline earth metal cation, preferably selected from the group consisting of sodium and potassium.

The compound of the invention of formula (I) may be prepared from (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium bromide or methanesulfonate of formula (II) as reported in Examples 1.1 and 1.2, wherein X is bromide or methanesulfonate respectively, obtained according e.g. to the procedures described in WO 2014/095700, or from propionate as reported in Example 1.3, by dissolution in an aqueous or organic solvent, such as preferably water, and addition with a solution of sodium or potassium hemi-pamoate salt of formula (III) in an appropriate aqueous or organic solvent, preferably water, under mechanical stirring.

Similarly the salt of the compound of the invention of formula (I) can be obtained using any water soluble salt of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium.

Compounds used as starting materials or intermediates may be commercially available, their preparation may be specifically described in the literature or they may be prepared according to methods available in the literature and well known to the person skilled in the art.

The process described is particularly advantageous as it is susceptible of being properly modulated, through any proper variant known to the skilled person, so as to obtain the desired compounds of the invention.

The invention is further illustrated by the following examples.

### EXAMPLES

### Nuclear Magnetic Resonance Spectroscopy (1H NMR)

All the 1H NMR spectra were performed on a Bruker AVANCE III HD 600 spectrometer operating at 600 MHz (proton frequency). The spectrometer was equipped with a 5 mm TCI INVERSE TRIPLE RESONANCE CRYOPROBE H-C/N-D-0.5-Z ATMA. The probe is fitted with an actively shielded single axis Z-gradient and allowed simultaneous decoupling on multiple X-nuclei such as 13C and 15N as well as automatic tuning and matching.

### Example 1

### Example 1.1

### (2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium hemi-pamoate

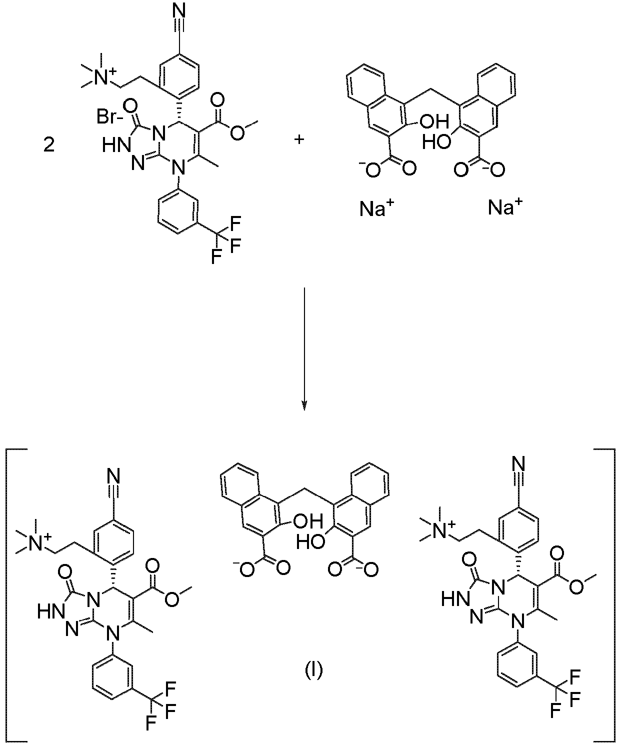

(2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium bromide (500 mg; 0.8 mmol), prepared as described in WO 2014/095700, was dissolved in water (25 ml) and added with a solution of sodium pamoate (174 mg, 0.4 mmol) in water (8 ml) under mechanical stirring, obtaining hemi-pamoate salt as a solid. Stoichiometric ratio between (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium cation and pamoate di-anion is 2:1 is confirmed by NMR.

1H NMR (400 MHz, DMSO-d6) δ ppm 11.32 (s, 1 H) 8.19 (d, J=8.55 Hz, 1 H) 8.12 (s, 2 H) 7.87 - 7.97 (m, 2 H) 7.69 - 7.87 (m, 4 H) 7.59 (d, J=7.89 Hz, 1 H) 7.08 (t, J=7.12 Hz, 1 H) 6.97 (t, J=6.91 Hz, 1 H) 6.26 (s, 1 H) 4.65 (s, 1 H) 3.92 - 4.02 (m, 1 H) 3.64 - 3.79 (m, 2 H) 3.53 (s, 3 H) 3.33 - 3.46 (m, 1 H) 3.21 (s, 9 H) 2.16 (s, 3 H)

### Example 1.2

### (2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium hemi-pamoate

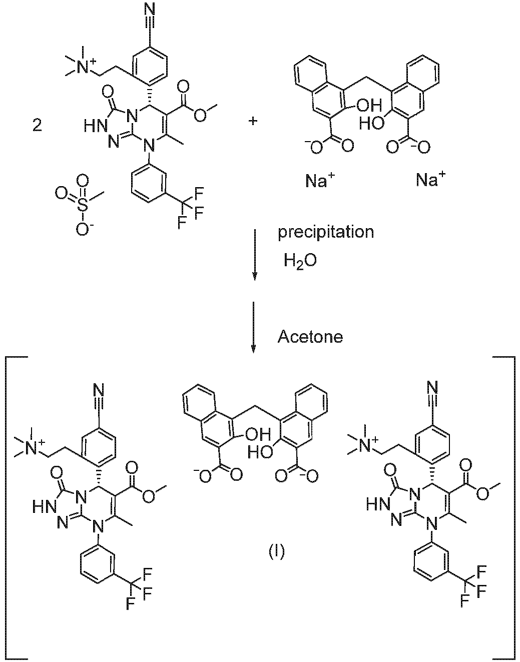

(2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium methanesulfonate (19.1 g, 30 mmol), prepared as described in WO 2014/095700, was dissolved in water (1000 ml) and added with a solution of sodium pamoate (6.5 g, 15 mmol) in water (250 ml) under mechanical stirring, obtaining the hemi-pamoate salt as a solid. The solid was filtered and washed with water (50 ml), then the wet solid was transferred into a flask and added with acetone (500 ml). The slurry was stirred at 50°C for 2 h, then refrigerated and left at 5°C overnight. The solid was filtered, washed with acetone and dried under vacuum at 45°C. 18 g of a solid was obtained. The compound was identical to that obtained starting from the bromide salt of Example 1.

Similarly the hemi-pamoate salt can be obtained using any other water soluble salt of 2-{ 5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium in place of the bromide salt or of the methanesulfonate salt.

A further soluble salt of (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium may be the propionate, obtained as here below described.

### Example 1.3

### (2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium propionate - Process a

To a solution of 2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium methanesulfonate (1 g, 1.57 mmol) in water (25 mL) was added 5 mL of washed Amberlyst A26 OH resin and the mixture tumbled at RT for 1H. The reaction mixture was filtered and the resin washed with water (25 mL). The combined filtrate and washings were freeze-dried to give the betaine product as a solid (738 mg, 87%).

1H NMR (400 MHz, d6-DMSO) δ 7.93 (1H, s), 7.89-7.82 (1H, m), 7.82-7.73 (3H, m), 7.68 (1H, dd, J = 8.3, 1.7 Hz), 7.53 (1H, d, J = 7.9 Hz), 5.98 (1H, s), 4.67 (1H, t, J = 11.1 Hz), 3.80 (1H, td, J = 12.4, 3.5 Hz), 3.59 (1H, td, J = 12.0, 6.1 Hz), 3.51 (3H, s), 3.32 (9H, br s), 3.27-3.22 (1H, m), 2.14 (3H, s).

To a solution of the betaine (738 mg, 1.37 mmol) in water (5 mL) was added propionic acid (0.11 mL, 1.50 mmol) and the mixture stirred at RT for 5 min. The reaction mixture was freeze-dried to give the product as a solid (840 mg, 100%).

1H NMR (400 MHz, d6-DMSO) δ 8.1 (1H, s), 7.95-7.87 (2H, m), 7.85-7.78 (2H, m), 7.75 (1H, dd, J = 8.0, 1.8 Hz), 7.68 (1H, d, J = 8.3 Hz), 6.22 (1H, s), 4.13-4.01 (1H, m), 3.78-3.65 (2H, m), 3.52 (3H, s), 3.42-3.31 (1H, m), 3.21 (9H, s), 2.16 (3H, s), 1.83 (2H, q, J = 7.6 Hz), 0.86 (3H, t, J = 7.6 Hz).

### (2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium propionate - Process b

To a solution of 2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium methanesulfonate (32.58 g, 51.2 mmol) in water (120 mL) was added 165 mL of washed Amberlyst A26 OH resin (~150 mmol eq OH) and the mixture tumbled at RT for 1H. The reaction mixture was filtered and the resin washed with water (~250 mL). To the combined filtrate and washings was added propionic acid (4 mL, 53.7 mmol) and the mixture stirred at RT for 5 min. The solution was freeze-dried to give the product as a solid (26.1 g, 83%).

1H NMR indicated that this sample contained 1.15 equivalents of propionic acid. 1H NMR (400 MHz, d6-DMSO) δ 8.1 (1H, s), 7.98-7.86 (2H, m), 7.86-7.78 (2H, m), 7.75 (1H, dd, J = 8.0, 1.8 Hz), 7.68 (1H, d, J = 8.3 Hz), 6.22 (1H, s), 4.12-4.00 (1H, m), 3.79-3.65 (2H, m), 3.53 (3H, s), 3.42-3.30 (1H, m), 3.21 (9H, s), 2.16 (3H, s), 1.85 (2H, q, J = 7.6 Hz), 0.86 (3H, t, J = 7.6 Hz).

### Example 2

### Biological Assay

The efficacy of different salts of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium was evaluated in the *in vivo* model of lung injury induced by human neutrophil elastase (HNE).

In addition, the effects of different (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium salts on lung function parameters were also assessed in rats by head-out plethysmography in order to compare the potential local adverse effects of each salt of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium.

### Example 2.1/Comparative

### HNE-induced lung injury assay

Male Sprague Dawley rats were dosed intratracheally (i.t.) with either vehicle (1% tween80 in saline), (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium methanesulfonate salt or (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate salt 3 hours prior to phosphate buffered saline (PBS) (control group, PBS alone treated animals ) or HNE intratracheal administration (100U/rat). Then, one hour after the PBS or HNE administration animals were sacrificed and bronchoalveolar lavage (BAL) was performed in order to assess the HNE-induced lung injury measured as haemoglobin concentration. BAL fluid samples were centrifuged at 800 g for 15 min at 4°C. The supernatant was collected, and the pellet was re-suspended in 3 mL of distilled water. A standard curve with known volumes of lysated blood cells was made from a stem solution of lysated blood cells. 150 µL of standards and samples were transferred in duplicate to a 96-well plate and the OD measured at 412 nm. Percentage of compound efficacy (assessed as inhibition of HNE-induced haemoglobin content in BALF) was calculated following this formula: 100- [(mean of haemoglobin concentration of test compound treated rats exposed to HNE)-(mean of haemoglobin concentration of vehicle treated rats exposed to PBS)]/[(mean of haemoglobin concentration of vehicle treated rats exposed to HNE)-( mean of haemoglobin concentration of vehicle treated rats exposed to PBS)]x100.

### Results

In this model, HNE i.t. challenge induces a significant increase of haemoglobin content when compared to control group (0 g/dL for control group and 0.19 g/dL for HNE group, p<0.001).

(2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium methanesulfonate and hemi-pamoate salts administered i.t. as wet formulation at three different doses (3, 30 and 300 µg/kg for methanesulfonate and hemi-pamoate salts) showed a dose-dependent inhibition of BAL fluid haemoglobin content. Specifically, the methanesulfonate salt showed an inhibition ranging from 19% at 3 µg/kg, 30 % at 30 µg/kg and 73% at 300 µg/kg (p<0.001) when compared to the HNE-treated vehicle control. Similarly, the hemi-pamoate salt induced a BAL fluid haemoglobin content reduction ranging from 30% at 3 µg/kg, 43% at 30 µg/kg (p<0.05) and 80% at 300 µg/kg (p<0.001) in comparison with the HNE-treated vehicle control.

These data demonstrate that the two salts of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium are able to inhibit HNE in vivo, and surprisingly despite the lower solubility, the hemi-pamoate salt shows a slightly superior efficacy than the methanesulfonate salt in this model.

In addition, the efficacy of '(2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium hemi-pamoate salt in this model was maintained when the compound was administered as dry powder formulation. Specifically, i.t. administration of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium hemi-pamoate salt given as dry powder resulted in a dose-dependent inhibition of BAL fluid haemoglobin levels ranging from 4% at 30 µg/kg, 54% at 300 µg/kg (p<0.05) and 60% at 600 µg/kg (p<0.01) when compared to the HNE-treated vehicle (lactose) control group.

### Solubility in water of

### (2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium methanesulfonate

200 mg of (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium methanesulfonate were placed in 1 ml of water at RT. The complete dissolution of the material was visually observed.

The visual observation of an instantaneous dissolution was index that the methanesulfonate of formula (I) has a solubility >200 mg/ml.

### Solubility in water of

### (2-{5-Cyano-2-[(R)-6-methoxycarbonyl-7-methyl-3-oxo-8-(3-trifluoromethylphenyl)-2,3,5,8-tetrahydro-[1,2,4]triazolo[4,3-a]pyrimidin-5-yl]-phenyl}-ethyl)-trimethyl-ammonium hemi-pamoate

A saturated solution of hemi-pamoate salt in water at RT was prepared, with a theoretical concentration of about 2 mg/ml. The solid in excess was filtered out and the content of the solution was quantified by LC/UV at different check points until 24 hours.

The hemi-pamoate salt has a solubility of 0.17 mg/ml.

The results of the solubility tests are summarized in the following **Table 1:**

| **Salt** | **Solubility (mg/ml)** |
|---|---|
| Methanesulfonate | >200 |
| Hemi-pamoate | 0.17 |

The above **Table 1** clearly shows a significantly higher solubility of the methanesulfonate salt with respect to the hemi-pamoate salt.

The results of the HNE-induced lung injury assay are summarized in the following **Table 2:**

| **Salt** | **% Inhibition at 3 µg/Kg** | **% Inhibition at 30 µg/Kg** | **% Inhibition at 300 µg/Kg** |
|---|---|---|---|
| Methanesulfonate | 19 | 30 | 73 |
| Hemi-pamoate | 30 | 43 | 80 |

The above **Table 2** clearly shows that both the hemi-pamoate and methanesulfonate salts are able to inhibit HNE in vivo, but, despite the lower solubility, the hemi-pamoate salt shows a superior activity in the above described pharmacodynamic model, with respect to the methanesulfonate salt.

### Example 2.2/Comparative

### Head-out plethysmography (HOP) assay

Male Wistar rats were dosed by the snout-only inhalation route with (2-{ 5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium methanesulfonate salt, (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium hemi-pamoate salt and (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium propionate salt (whose synthesis is described in example 1.3), (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium bromide salt (whose synthesis is described in WO 2014/095700) or lactose given as dry powder formulations. The duration of aerosol exposure was of 60 minutes. On the day of dosing, animals were placed into the plethysmograph tubes for at least 30 minutes prior to dosing and respiratory parameters: respiratory rate, tidal volume, and PenH were continuously recorded for at least 30 minutes pre-dose, 60 minutes during dosing (exposure) and 90 minutes post-exposure. The respiratory parameters were recorded every minute for a total period of 3 hours using the EMMS eDacq system (PLY231, EMMS, Bordon, United Kingdom). The effect of the test compounds on different lung function parameters was measured as % of change versus vehicle (lactose) group and was reported at peak effect (i.e. the highest effect observed).

### Results

A single inhaled administration of two doses of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium methanesulfonate salt (0.6 and 6 mg/kg) as dry powder produced statistically significant changes in all three respiratory parameters analysed and these effects were mainly observed during exposure. Specifically, a significant increase of respiratory rate was observed at both doses during inhalation with a peak effect of 32% of increase observed at the dose of 0.6 mg/kg and a maximum increase of 54% induced by the dose of 6 mg/kg. The increase of respiratory rate observed during inhalation of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium methanesulfonate salt was associated with a significant reduction of tidal volume of 35% and 39% (peak effect at the doses of 0.6 and 6 mg/kg, respectively) and a significant and dose-dependent increase of PenH of about 8-fold for the low dose and 16-fold for the high dose compared to vehicle group.

Similar effects were observed with (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium bromide salt (0.55 and 5.5 mg/kg), which produced statistically significant reduction in tidal volume during inhalation, with a maximum reduction of 38% for the high dose group.

This decrease in tidal volume also coincided with a statistically significant increase in PenH area of about 8-fold for the low dose and 20-fold for the high dose compared to vehicle, and a significant increase in respiratory rate again during inhalation with the low dose reaching a maximum effect of 59% compared to vehicle. Likewise, (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium propionate salt administered via single snout-only inhalation at 0.6 mg/kg (low dose) and 6 mg/kg (high dose) produced statistically significant reduced tidal volume at both doses when compared to the vehicle control with a maximum decrease of 37% in the high dose group as well as a statistically significant increase in respiratory rate at the low dose reaching a maximum effect of 65%. These changes were associated with a significant increase in PenH area of about 7-fold for the low dose and 18-fold for the high dose.

The peak effects for all three (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl }-ethyl)-trimethyl-ammonium salts were observed after 20-50 minutes of compound inhalation with return to baseline values occurring immediately after dosing.

Conversely, (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate salt administered via single snout-only inhalation at 0.7 and 7 mg/kg as dry powder did not alter any lung function parameters either during or after dosing.

The results of the Head-out plethysmography (HOP) assay are summarized in the following **Table 3:**

| **Salt** | **% of Reduction of tidal volume** |
|---|---|
| Methanesulfonate | 35-39 |
| Bromide | 38 |
| Propionate | 37-65 |
| Hemi-pamoate | No variation of any lung function parameters |

These data demonstrate that conversely to the other salts, the hemi-pamoate salt does not affect any lung function parameters, suggesting that this salt is particularly suitable for administration, also in terms of patients tolerability and local adverse effects profile.

## Claims

1. The compound (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium hemi-pamoate of formula (I)

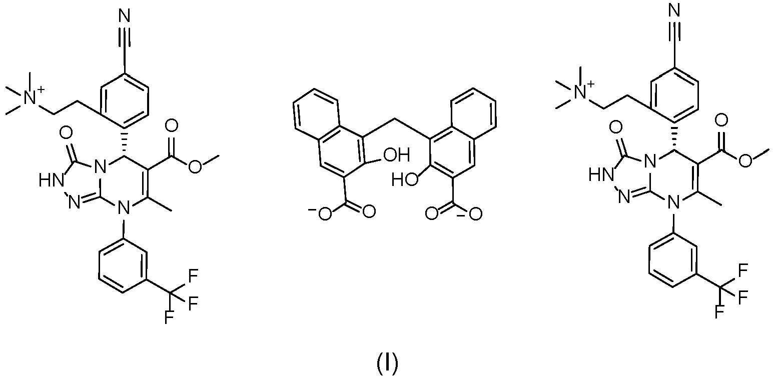

2. A pharmaceutical composition comprising the compound of formula (I) of claim 1 and one or more pharmaceutically acceptable carriers and/or excipients.

3. A pharmaceutical composition according to claim 2, formulated in form of a dry powder.

4. The compound of formula (I) of claim 1, for use as a medicament.

5. The composition according to claims 2 and 3, for use as a medicament.

6. The compound of formula (I) of claim 1, for use in the prevention and/or treatment of an inflammatory or obstructive respiratory disease.

7. The pharmaceutical composition according to claims 2 and 3, for use in the prevention and/or treatment of an inflammatory or obstructive respiratory disease.

8. The compound for use in the prevention and/or treatment of an inflammatory or obstructive respiratory disease according to claim 6, wherein the inflammatory or obstructive respiratory diseases are selected from : asthma, chronic obstructive pulmonary disease (COPD), bronchiectasis, chronic bronchitis, lung fibrosis, idiopathic pulmonary fibrosis, pneumonia, acute respiratory distress syndrome (ARDS), pulmonary emphysema, smoking-induced emphysema and cystic fibrosis.

9. The pharmaceutical composition for use for the prevention and/or treatment of an inflammatory or obstructive respiratory disease according to claim 7, wherein the inflammatory or obstructive respiratory diseases are selected from asthma, chronic obstructive pulmonary disease (COPD), bronchiectasis, chronic bronchitis, lung fibrosis, idiopathic pulmonary fibrosis, pneumonia, acute respiratory distress syndrome (ARDS), pulmonary emphysema, smoking-induced emphysema and cystic fibrosis.

10. A process for the preparation of the compound of formula (I) according to claim 1, by reacting the triazolone derivative of formula (II) with a pamoate salt of formula (III) 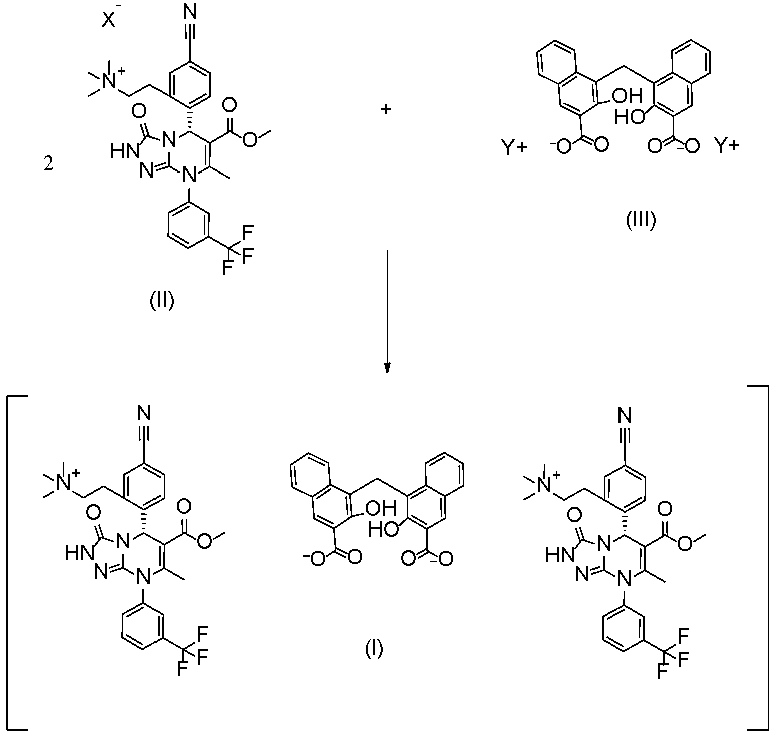 said process comprising the steps of:
1) dissolving in water a water soluble salt of (2-{5-Cyano-2-[(R)-6-methoxycarbonyl-7-methyl-3-oxo-8-(3-trifluoromethyl-phenyl)-2,3,5,8-tetrahydro-[1,2,4]triazolo[4,3-a]pyrimidin-5-yl]-phenyl}-ethyl)-trimethyl-ammonium of formula (II): 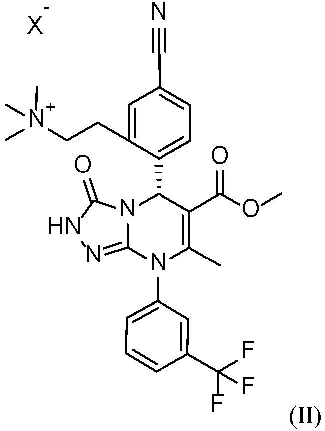 wherein X⁻ is an organic or inorganic anion, and
2) adding a solution of a pamoate salt of formula (III) 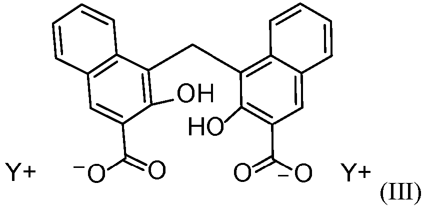 wherein Y⁺ is an alkaline or alkaline earth metal cation.

11. A process according to claim 10, wherein the molar ratio between the compound of formula (II) and the pamoate salt of formula (III) is 2:1.

12. A process according to claims 10 or 11, wherein X⁻ is bromide.

13. A process according to claims 10 or 11, wherein X⁻ is methanesulfonate.

14. A process according to claims 10 or 11, wherein X⁻ is propionate.

15. A process according to claims 10 to 14, wherein Y⁺ is sodium or potassium.

## Patentansprüche

1. Verbindung (2-{5-Cyano-2-[(R)-6-methoxycarbonyl-7-methyl-3-oxo-8-(3-trifluormethyl-phenyl)-2,3,5,8-tetrahydro-[1,2,4]triazolo[4,3-a]pyrimidin-5-yl]-phenyl}-ethyl)-trimethyl-ammonium-hemi-pamoat der Formel (I)

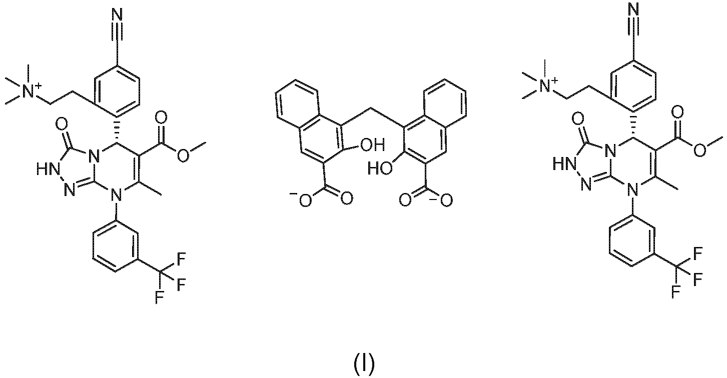

2. Pharmazeutische Zusammensetzung, umfassend die Verbindung der Formel (I) nach Anspruch 1 und einen oder mehrere pharmazeutisch unbedenkliche Träger und/oder Hilfsstoffe.

3. Pharmazeutische Zusammensetzung nach Anspruch 2, formuliert in Form eines trockenen Pulvers.

4. Verbindung der Formel (I) nach Anspruch 1 zur Verwendung als Medikament.

5. Zusammensetzung nach Anspruch 2 und 3 zur Verwendung als Arzneimittel.

6. Verbindung der Formel (I) nach Anspruch 1 zur Verwendung bei der Vorbeugung und/oder Behandlung einer entzündlichen oder obstruktiven Atemwegserkrankung.

7. Pharmazeutische Zusammensetzung nach den Ansprüchen 2 und 3 zur Verwendung bei der Vorbeugung und/oder Behandlung einer entzündlichen oder obstruktiven Atemwegserkrankung.

8. Verbindung zur Verwendung bei der Vorbeugung und/oder Behandlung einer entzündlichen oder obstruktiven Atemwegserkrankung nach Anspruch 6, wobei die entzündlichen oder obstruktiven Atemwegserkrankungen ausgewählt sind aus: Asthma, chronisch obstruktiver Lungenerkrankung (COPD), Bronchiektasie, chronischer Bronchitis, Lungenfibrose, idiopathischer Lungenfibrose, Lungenentzündung, akutem Atemnotsyndrom (ARDS), Lungenemphysem, rauchinduziertem Emphysem und zystischer Fibrose.

9. Pharmazeutische Zusammensetzung zur Verwendung für die Vorbeugung und/oder Behandlung einer entzündlichen oder obstruktiven Atemwegserkrankung nach Anspruch 7, wobei die entzündlichen oder obstruktiven Atemwegserkrankungen ausgewählt sind aus Asthma, chronisch obstruktiver Lungenerkrankung (COPD), Bronchiektasie, chronischer Bronchitis, Lungenfibrose, idiopathischer Lungenfibrose, Lungenentzündung, akutem Atemnotsyndrom (ARDS), Lungenemphysem, rauchinduziertem Emphysem und zystischer Fibrose.

10. Prozess zur Herstellung der Verbindung der Formel (I) nach Anspruch 1, durch Umsetzen des Triazolonderivats der Formel (II) mit einem Pamoatsalz der Formel (III) 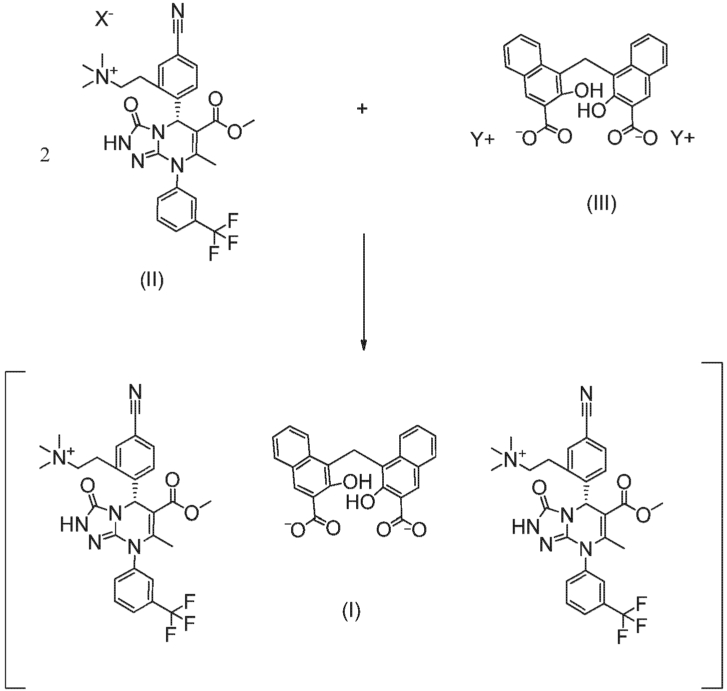 das Verfahren umfassend die folgenden Schritte:
1) Lösen eines wasserlöslichen Salzes von (2-{5-Cyano-2-[(R)-6-methoxycarbonyl-7-methyl-3-oxo-8-(3-trifluormethyl-phenyl)-2,3,5,8-tetrahydro-[1,2,4]triazolo[4,3-a]pyrimidin-5-yl]-phenyl}-ethyl)-trimethyl-ammonium der Formel (II) in Wasser: 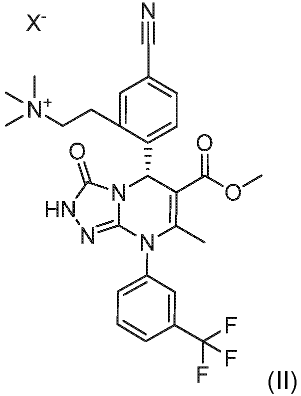 worin X⁻ ein organisches oder anorganisches Anion ist, und
2) Zugeben einer Lösung eines Pamoatsalzes der Formel (III) 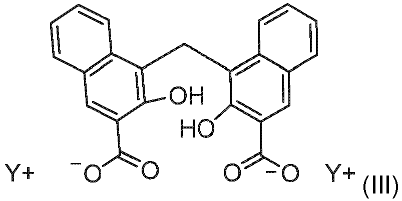 worin Y⁺ ein Alkali- oder Erdalkalimetallkation ist.

11. Prozess nach Anspruch 10, wobei das molare Verhältnis zwischen der Verbindung der Formel (II) und dem Pamoatsalz der Formel (III) 2:1 beträgt.

12. Prozess nach Anspruch 10 oder 11, wobei X⁻ Bromid ist.

13. Prozess nach Anspruch 10 oder 11, wobei X⁻ Methansulfonat ist.

14. Prozess nach Anspruch 10 oder 11, wobei X⁻ Propionat ist.

15. Prozess nach einem der Ansprüche 10 bis 14, wobei Y⁺ Natrium oder Kalium ist.

## Revendications

1. Composé (2-{5-Cyano-2-[(R)-6-méthoxycarbonyl-7-méthyl-3-oxo-8-(3-trifluorométhyl-phényl)-2,3,5,8-tétrahydro-[1,2,4]triazolo[4,3-a]pyrimidin-5-yl]-phényl}-éthyl)-hémi-pamoate de triméthyl-ammonium de formule (I)

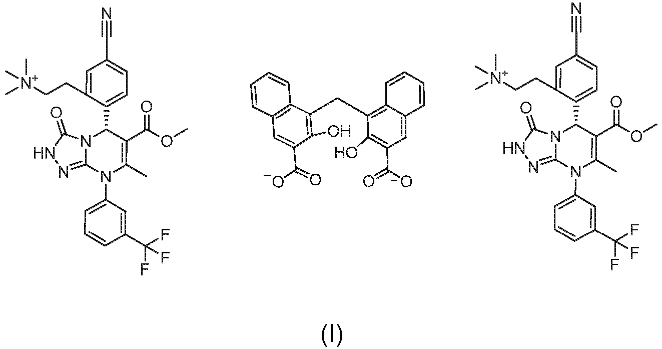

2. Composition pharmaceutique comprenant le composé de formule (I) selon la revendication 1 et un ou plusieurs supports et/ou excipients pharmaceutiquement acceptables.

3. Composition pharmaceutique selon la revendication 2, formulée sous forme de poudre sèche.

4. Composé de formule (I) selon la revendication 1, pour une utilisation en tant que médicament.

5. Composition selon les revendications 2 et 3, pour une utilisation en tant que médicament.

6. Composé de formule (I) selon la revendication 1, pour une utilisation dans la prévention et/ou le traitement d'une maladie respiratoire inflammatoire ou obstructive.

7. Composition pharmaceutique selon les revendications 2 et 3, pour une utilisation dans la prévention et/ou le traitement d'une maladie respiratoire inflammatoire ou obstructive.

8. Composé pour une utilisation dans la prévention et/ou le traitement d'une maladie respiratoire inflammatoire ou obstructive selon la revendication 6, dans lequel les maladies respiratoires inflammatoires ou obstructives sont choisies parmi : asthme, broncho-pneumopathie chronique obstructive (BPCO), bronchectasie, bronchite chronique, fibrose pulmonaire, fibrose pulmonaire idiopathique, pneumonie, syndrome de détresse respiratoire aiguë (SDRA), emphysème pulmonaire, emphysème induit par le tabagisme et fibrose kystique.

9. Composition pharmaceutique pour une utilisation dans la prévention et/ou le traitement d'une maladie respiratoire inflammatoire ou obstructive selon la revendication 7, dans laquelle les maladies respiratoires inflammatoires ou obstructives sont choisies parmi l'asthme, la broncho-pneumopathie chronique obstructive (BPCO), la bronchectasie, la bronchite chronique, la fibrose pulmonaire, la fibrose pulmonaire idiopathique, la pneumonie, le syndrome de détresse respiratoire aiguë (SDRA), l'emphysème pulmonaire, l'emphysème induit par le tabagisme et la fibrise kystique.

10. Procédé de préparation du composé de formule (I) selon la revendication 1, consistant à faire réagir le dérivé de triazolone de formule (II) avec un sel de pamoate de formule (III) 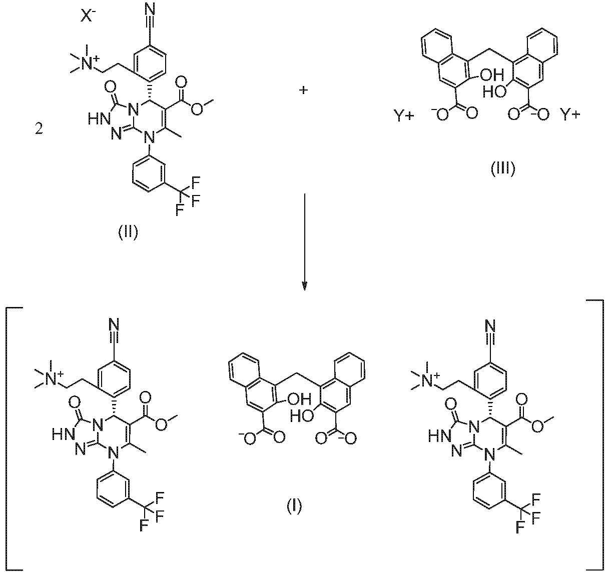 ledit procédé comprend les étapes consistant à :
1) dissoudre dans de l'eau un sel hydrosoluble de (2-{5-Cyano-2-[(R)-6-méthoxycarbonyl-7-méthyl-3-oxo-8-(3-trifluorométhyl-phényl)-2,3,5,8-tétrahydro-[1,2,4]triazolo[4,3-a]pyrimidin-5-yl]-phényl}-éthyl)-triméthyl-ammonium de formule (II) : 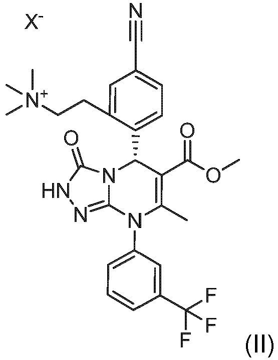 où X⁻ est un anion organique ou inorganique, et
2) ajouter une solution d'un sel de pamoate de formule (III) 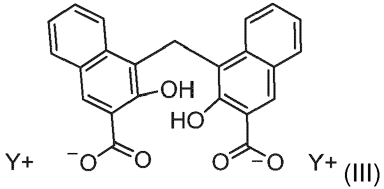 où Y⁺ est un cation métallique alcalin ou alcalino-terreux.

11. Procédé selon la revendication 10, dans lequel le rapport molaire entre le composé de formule (II) et le sel de pamoate de formule (III) est de 2:1.

12. Procédé selon les revendications 10 ou 11, dans lequel X⁻ est un bromure.

13. Procédé selon les revendications 10 ou 11, dans lequel X⁻ est un méthanesulfonate.

14. Procédé selon les revendications 10 ou 11, dans lequel X⁻ est un propionate.

15. Procédé selon les revendications 10 à 14, dans lequel Y⁺ est du sodium ou du potassium.
